# FASCICULE DE BREVET EUROPEEN

(11) **EP 4 180 029 B1**
(45) Date de publication et mention de la délivrance du brevet: **03.04.2024**
(21) Numéro de dépôt: 22207160.7
(22) Date de dépôt: 14.11.2022
(51) Int. Cl.: A61K 8/64, A61K 8/9706, A61K 8/9789, A61Q 19/00

(54) **COMPOSITION POUR L'AMÉLIORATION DU BIEN-ÊTRE CUTANÉE**
ZUSAMMENSETZUNG ZUR VERBESSERUNG DES WOHLBEFINDENS DER HAUT
COMPOSITION FOR THE IMPROVEMENT OF SKIN WELL-BEING

(30) Priorité: 15.11.2021 FR 2112027
(43) Date de publication de la demande: 17.05.2023
(73) Titulaire: Scherk GmbH, 10179 Berlin (DE)
(72) Inventeur: Henssen, Peter, Berlin (DE); Saguet, Thibaut, Olivet (FR)
(74) Mandataire: Cabinet Célanie

(56) Documents cités:
- FR-A1- 2 829 694
- DATABASE GNPD [Online] MINTEL; 1 août 2016 (2016-08-01), anonymous: "Crème Originelle Preventative Anti-Ageing Skincare Dry Skin", XP055937665, Database accession no. 4174595
- DATABASE GNPD [Online] MINTEL; 2 juillet 2012 (2012-07-02), anonymous: "Melon & Fruit Children Delicate Moisture Cream", XP055937667, Database accession no. 1825043

## Description

### DOMAINE DE L'INVENTION

Le secteur technique de la présente invention concerne les compositions pour l'amélioration du bien-être cutanée et plus particulièrement les compositions cosmétiques comprenant des principes actifs naturels tels que l'extrait de Caulerpa lentillifera, des protéines de riz hydrolysées, l'extrait d'Hibiscus esculentus Moench et l'extrait de Luffa cylindrica.

### ETAT DE LA TECHNIQUE

A l'interface avec l'air, la peau est la cible de plusieurs stress environnementaux, tels que la pollution et les rayonnements UV. Les polluants atmosphériques pénètrent dans la peau directement ou indirectement et induisent plusieurs changements biochimiques comme une augmentation de la production des formes réactives de l'oxygène, mais aussi une diminution de la prolifération cellulaire et des antioxydants. Cliniquement, les effets des polluants entrainent une exacerbation des processus de vieillissement cutané, des symptômes de maladies inflammatoires et la dérégulation de l'hydratation cutanée. De plus, La combinaison des rayonnements UV et des polluants exacerbe les effets biochimiques et cliniques des polluants atmosphériques.

Tous ces stress, auxquels la peau est sujette, entrainent donc une modification structurelle et un dérèglement de l'homéostasie cutanée. Le bien-être cutané vise à rétablir cette homéostasie en rendant à la peau sa souplesse et son élasticité.

Le bien-être cutané peut être apporté par des moyens physiques tels que des massages ou chimiques tels que des compositions cosmétiques ou dermatologiques.

Depuis ces deux dernières décennies, l'industrie cosmétique se tourne de plus en plus vers des principes actifs naturels et souvent extraits de plantes ou d'algues qui présentent des avantages comme le moindre coût de revient et la compatibilité biochimique avec la peau humaine.

L'algue *Caulerpa lentillifera* est une algue verte retrouvée sur la côte est africaine, en Asie, au niveau des iles du pacifique, en Australie, en Nouvelle-Zélande et aux Maldives. Elle est utilisée comme aliment dans des pays comme le Japon et les Philippines. En cosmétique, elle est connue pour améliorer la santé de la peau (CN105852128) et blanchir la peau (JP2015086187) ainsi que pour ses effets antiâge (JP2015086189) et antioxydants (TW201713353). Mintel GNPD Record ID 4174595 "Crème Originelle Preventative Anti-Ageing Skincare Dry Skin",aout 2016 divulgue une composition cosmétique comprenant de l'extrait de Caulerpa lentillifera, et des protéines de riz hydrolysées.

Le riz est connu pour fournir, par ces extraits d'hydrolysat peptidique tel que l'extrait d'Oryza sativa, des ingrédients cosmétiques. On sait de Sim et al., 2007 que l'hydrolysat peptidique de riz noir favorise la voie de biosynthèse d'acide hyaluronique des kératinocytes et de Chen et al., 2021 qu'il a des propriétés antioxydante et antiâge.

L'*Hibiscus esculentus* est un arbre poussant dans des zones tropicales et tempérées. L'extrait d'*Hibiscus esculentus Moench* est connu pour stimuler la production d'acide hyaluronique, l'hydratation de la peau et permet d'apporter un effet glissant à la peau. On sait du document US1011782 qu'un extrait de mucilage d'*Hibiscus esculentus* présente des propriétés lubrifiante et hydratante et du document FR2829694 qu'il présente des propriétés anti-radicalaire et antivieillissement.

*Luffa cylindrica* est une plante grimpante de la famille des Cucurbitaceae et est notamment utilisée comme aliment ou complément alimentaire. On sait du document WO03017966 que l'huile de *Luffa cylindrica* peut être utilisée dans une composition cosmétique permettant de relipider la peau.

Il n'existe à l'heure actuelle aucune composition comprenant en combinaison de l'extrait de *Caulerpa lentillifera,* des protéines de riz hydrolysées, de l'extrait d'*Hibiscus esculentus Moench* et de l'extrait de *Luffa cylindrica.*

### EXPOSE DE L'INVENTION

L'invention concerne donc une composition cosmétique comprenant à titre de principe actif de l'extrait de Caulerpa lentillifera, des protéines de riz hydrolysées, de l'extrait d'*Hibiscus* esculentus Moench et de l'extrait de Luffa cylindrica, le reste de la composition comprenant des ingrédients généralement admissibles dans le domaine cosmétique.

Avantageusement, la composition comprend entre 0,05% et 8% en masse d'extrait de Caulerpa lentillifera, entre 0,001% et 1% en masse de protéines de riz hydrolysées, entre 0,05% et 8% en masse d'extrait d*'*Hibiscus esculentus et entre 0,001% et 5% d'extrait de Luffa cylindrica.

Selon un mode de réalisation de l'invention, la composition comprend également un ou plusieurs agents antioxydants.

Selon un autre mode de réalisation de l'invention, la composition comprend également de l'extrait de feuille de thé.

Avantageusement, la composition comprend entre 1% et 3% d'extrait de feuille de thé.

Selon encore un autre mode de réalisation de l'invention, la composition se présente sous la forme d'une crème, d'un sérum, d'un gel, d'un baume ou d'une lotion.

Un avantage de la présente invention est qu'elle permet de maintenir une bonne homéostasie cutanée.

Un autre avantage encore de la présente invention est qu'elle permet de préserver et de rétablir l'intégrité de la barrière cutanée.

Un autre avantage encore de la présente invention est qu'elle permet d'augmenter la production de dopamine des kératinocytes.

Un autre avantage encore de la présente invention est qu'elle permet d'augmenter l'activité télomérase des kératinocytes.

Un autre avantage encore de la présente invention est qu'elle permet de diminuer les réactions inflammatoires de la peau.

Un autre avantage encore de la présente invention est qu'elle permet d'augmenter la viabilité cellulaire des cellules souches de peau.

D'autres caractéristiques, avantages et détails de l'invention seront mieux compris à la lecture du complément de description qui va suivre.

### DESCRIPTION DETAILLEE DE MODES DE REALISATION DE L'INVENTION

Comme évoqué précédemment, l'invention concerne une composition cosmétique comprenant à titre de principe actif de l'extrait de Caulerpa lentillifera, des protéines de riz hydrolysées, de l'extrait d'Hibiscus esculentus Moench et de l'extrait de Luffa cylindrica.

L'extrait de Caulerpa lentillifera (dénomination INCI : Caulerpa lentillifera extract) selon l'invention comprend en outre :
- des oligosaccharides,
- des minéraux tels que su sodium, du potassium, du calcium et du magnésium,
- des acides gras,
- des pigments tels que la siphoaxanthine et la siphoneine,
- de la caulerpényne,
- de la caulerpicine, et
- des acides aminés tels que l'acide glutamique, l'acide aspartique, l'arginine, laleucine, l'alanine, la lysine et la thréonine.

Les protéines de riz hydrolysées (dénomination INCI : Hydrolysed Rice Bran protein) selon l'invention comprenent en outre :
- des peptides,
- des protéines, et
- des acides aminés.

L'extrait d'Hibiscus esculentus Moench (Dénomination INCI : Hibiscus Esculentus Fruit Extract) selon l'invention comprend en outre :
- des phénols,
- des anthocyanidines,
- des aides aminés,
- des acides gras, et
- des polysaccharides tels que le D-galactose, de L-rhamnose, le L-arabinose et l'acide D-galacturonique.

L'extrait de Luffa cylindrica (dénomination INCI : Luffa Cylindrica Fruit/Leaf/Stem Extract) selon l'invention comprend en outre :
- des acides aminés,
- de la saponine,
- de la citrulline, et
- du xylane.

Au vu des propriétés individuelles de ces extraits, les inventeurs ont imaginé une composition combinant ces extraits en vue de traiter la peau et c'est ainsi qu'ils ont constaté avec surprise que la composition selon l'invention avait un effet bénéfique sur la peau. En effet, la composition présente un effet visible sur la préservation du bien-être cutané.

Les inventeurs ont alors analysé l'activité biologique de la composition selon l'invention sur des kératinocytes humains et des cellules souches issues de peau humaine et se sont rendus compte qu'elle induisait au niveau de la peau humaine :
- une stimulation de la production de dopamine par les kératinocytes,
- une protection des cellules souches issues de peau humaine, et
- une augmentation de l'activité télomérase des kératinocytes.

Pour analyser l'effet sur la production de dopamine par les kératinocytes, les inventeurs ont testé l'effet d'une composition cosmétique P1 comprenant 1,5% en masse de la composition selon l'invention. Le reste de la composition P1 est constitué par le milieu de culture utilisé pour la culture des kératinocytes.

La dopamine est un neurotransmetteur jouant un rôle régulateur important dans la sensation de bien-être. Elle agit sur les cellules par l'intermédiaire des récepteurs dopaminergiques de type D1 et D2. Ainsi, stimuler la production de dopamine des cellules cutanées permet de favoriser les processus biochimiques du métabolisme et de diminuer les réactions inflammatoires favorisées notamment par le stress et la tension.

La dopamine est également impliquée dans le placebome qui correspond à un ensemble de gênes et de protéines actifs sur les mécanismes de réponse biologique à l'effet placebo. L'activation du placebome permet en outre une réparation automatique de la barrière cutanée.

Les inventeurs ont testé l'effet de la composition P1 sur des kératinocytes humains normaux mis en culture monocouche. Les kératinocytes ont été mis à incuber pendant 24 heures dans un milieu de culture comprenant la composition P1. La quantité de dopamine libérée dans le milieu de culture a ensuite été mesurée à l'aide d'un kit ELISA adapté. Les mesures ont ensuite été normalisées par rapport à la quantité de protéines du lysat cellulaire en utilisant la méthode de Bradford.

Les résultats montrent que la composition P1 testée permet d'augmenter la quantité de dopamine de 42,6%.

Les inventeurs ont également testé les effets d'une composition comprenant 100% en masse de la composition selon l'invention et sont arrivés au même résultat. De la même façon, des compositions cosmétiques comprenant à titre de principe actif entre 0,05% et 8% en masse d'extrait de Caulerpa lentillifera, entre 0,001% et 1% en masse de protéines de riz hydrolysées, entre 0,05% et 8% en masse d'extrait d'Hibiscus esculentus et entre 0,001% et 5% d'extrait de Luffa cylindrica permettent également d'augmenter la production de dopamine.

Ainsi, la composition selon l'invention permet d'augmenter la production de dopamine par les kératinocytes, ce qui a notamment pour effet de favoriser la réparation de la barrière cutanée, de régénérer la peau, d'engendrer des sensations de bien-être et de diminuer les réactions inflammatoires de la peau.

Pour analyser l'effet sur la protection des cellules issues de peau humaine, les inventeurs ont testé l'effet de la composition cosmétique P1 comprenant 1,5% en masse de la composition selon l'invention.

Les cellules souches sont des cellules indifférenciées capables à la fois de générer des cellules spécialisées par différentiation cellulaire et de renouveler la réserve de cellules souches. Elles jouent un rôle central dans le développement des organismes, dans le maintien de leur intégrité et permettent également de réparer et de régénérer la peau.

Les inventeurs ont testé l'effet de la composition sur des cellules souches issues de peau humaine mises en culture monocouche. Les cellules souches ont été mises à incuber 24 heures dans un milieu de culture adapté puis irradiées par des UVB selon une dose de 30 mJ/cm². Elles ont ensuite été incubées pendant 6 jours à 37°C en présence ou en absence de la composition.

La viabilité cellulaire a ensuite été mesurée en utilisant la méthode du bleu Alamar.

Les résultats montrent que la composition P1 permet d'augmenter de 51,2% la viabilité des cellules souches issues de peau humaine.

Les inventeurs ont également testé les effets d'une composition comprenant 100% en masse de la composition selon l'invention et sont arrivés au même résultat. De la même façon, des compositions cosmétiques comprenant à titre de principe actif entre 0,05% et 8% en masse d'extrait de Caulerpa lentillifera, entre 0,001% et 1% en masse de protéines de riz hydrolysées, entre 0,05% et 8% en masse d'extrait d'Hibiscus esculentus et entre 0,001% et 5% d'extrait de Luffa cylindrica permettent également d'augmenter viabilité des cellules souches.

Ainsi, la composition selon l'invention permet d'augmenter la viabilité des cellules souches issues de peau humaine ce qui a notamment pour effet de régénérer, de réparer, d'assurer le bon renouvellement de la peau et de la protéger.

Pour analyser l'effet sur l'activité télomérase des kératinocytes, les inventeurs ont testé l'effet de la composition cosmétique P1 comprenant 1,5% en masse de la composition selon l'invention, l'effet de la composition cosmétique P2 comprenant 0,375% en masse de la composition selon l'invention et l'effet de la composition cosmétique P3 comprenant 0,75% en masse de la composition selon l'invention.

Les télomères sont des séquences d'ADN répétitives situées à l'extrémité des chromosomes et permettent en outre de préserver l'intégrité du patrimoine génétique. A chaque réplication cellulaire la longueur des télomères diminue jusqu'à ce que les cellules deviennent sénescentes. Elles arrêtent alors de se diviser et de fonctionner normalement.

La télomérase est une enzyme qui à chaque réplication de l'ADN permet de limiter la diminution de la longueur des télomères. Ainsi, l'augmentation de l'activité télomérase des kératinocytes par exemple permet de préserver l'intégrité de la peau.

Les inventeurs ont testé l'effet des compositions P1 à P3 sur des kératinocytes humains normaux mis en culture jusqu'à obtention d'une confluence de 75%. Les kératinocytes ont ensuite été incubés pendant 24 heures en présence ou en absence des compositions. L'activité télomérase a ensuite été mesurée en utilisant un kit spécifique et sensible par le biais d'une étape de PCR couplée à un dosage ELISA. Les mesures ont ensuite été normalisées par rapport à la quantité de protéines du lysat cellulaire en utilisant la méthode de Bradford.

Les résultats montrent que la composition P1 permet d'augmenter l'activité télomérase des kératinocytes de 16,9%. La composition P2 permet d'augmenter l'activité télomérase des kératinocytes de 29,1%. La composition P3 permet d'augmenter l'activité télomérase des kératinocytes de 24,7%.

Les inventeurs ont également testé les effets d'une composition comprenant 100% en masse de la composition selon l'invention et sont arrivés au même résultat. De la même façon des compositions cosmétiques comprenant à titre de principe actif entre 0,05% et 8% en masse d'extrait de *Caulerpa lentillifera,* entre 0,001% et 1% en masse de protéines de riz hydrolysées, entre 0,05% et 8% en masse d'extrait d'*Hibiscus esculentus* et entre 0,001% et 5% d'extrait de *Luffa cylindrica* permettent également d'augmenter l'activité télomérase.

Ainsi, la composition selon l'invention permet d'augmenter l'activité télomérase des kératinocytes ce qui a notamment pour effet préserver l'intégrité de la peau et d'assurer le bon fonctionnement de ces cellules.

La composition selon l'invention peut également comprendre de l'extrait de feuille de thé (dénomination INCI : Camellia sinensis Leaf extract). Par exemple, la composition selon l'invention comprend entre 1% et 3% en masse d'extrait de feuille de thé. Un tel extrait permet en outre de conférer à la composition des propriétés antimicrobienne, antioxydante et astringente.

La composition selon l'invention peut également comprendre des agents humectants comme la glycérine (dénomination INCI : Glycerin), le butylène glycol (Dénimination INCI : Butylen glycol), l'hyaluronate de sodium (dénomination INCI : Sodium hyaluronate) et le caprylyl glycol (dénomination INCI : Caprylyl glycol).

La composition selon l'invention peut également comprendre des agents émollients comme le sucrose distéarate (dénomination INCI : Sucrose distearate), l'alcool cétylique (dénomination INCI : Cetyl alcohol), le tridécyl stéarate (dénomination INCI : Tridecyl stéarate), le sucrose stéarate (dénomination INCI : sucrose stéarate), l'huile de tournesol (dénomination INCI : Helianthus annuus seed oil), l'alcool cétéarylique (dénomination INCI : Cetearyl alcohol), le dicaprylyl carbonate (dénomination INCI : Dicaprylyl carbonate), le stéarate de glycol (dénomination INCI : Glycetyl stearate), le caprylyl glycol (dénomination INCI : Caprylyl glycol), l'isoamyl cocoate (dénomination INCI : Isoamyl cocoate), le coco-caprylate/caprate (dénomination INCI : Coco-caprylate/caprate, le cétyl ricinoléate (dénomination INCI : cétyl ricinoléate), le citrate de stéarate de glycéride (dénomination INCI : Glyceryl stéarate citrate).

La composition selon l'invention peut également comprendre des agents émulsifiants comme le Polyglyceryl-4 diisostéarate / Polyhydroxystearate / Sebecate (dénomination INCI : Polyglyceryl-4 diisostearate / Polyhydroxystearate / Sebecate), la gomme xanthane (dénomination INCI : Xanthan gum), le stéarate de glycol (dénomination INCI : Glycetyl stéarate), du Candelilla / Jojoba / Rice bran polyglyceryl-3 esters, Glycéryl stéarate, alcool cétéarylique, Sodium stéaroyl lactylate (dénomination INCI : Candelilla / Jojoba / Rice bran polyglyceryl-3 esters, Glyceryl stéarate, Cetearyl alcohol, Sodium stearoyl lactylate), de la Lysolécithine / gomme de sclérotium rolfssii / Pullulan (dénomination INCI : Lysolecithin / Sclerotium gum / Pullulan), la gomme de sclérotium rolfssii (dénomination INCI : Sclerotium gum), du Polyglycéryl-6 distéarate / Jojoba esters / Polyglycéryl-3 Beeswax / alcool cétylique (dénomination INCI : Polyslycertyl-6 distearate / Jojoba esters / Polyglyceryl-3 beeswax / Cetyl alcohol), le citrate de stéarate de glycéride (dénomination INCI : Glyceryl stéarate citrate) et l'huile végétale (dénomination INCI : Olus oil).

La composition selon l'invention peut également comprendre des agents d'entretien et de protection de la peau comme la glycérine (dénomination INCI : Glycerin), le caprylic/capric triglycéride (dénomination INCI : Caprilic/Capric Tiglyceride), le sucrose distéarate (dénomination INCI : Sucrose distearate), le tridécyl stéarate (dénomination INCI : Tridecyl stéarate), l'huile de tournesol (dénomination INCI : Helianthus annuus seed oil), le sucrose stéarate (dénomination INCI : sucrose stéarate), la gomme xanthane (dénomination INCI : Xanthan gum), le butylène glycol (Dénimination INCI : Butylen glycol), la phytosphingosine (dénomination INCI : Phytosphingosine), le dicaprylyl carbonate (dénomination INCI : Dicaprylyl carbonate), la vitamine E (dénomination INCI : Tocopherol), le 1,2-hexanediol (dénomination INCI : 1,2-Hexanediol), le caprylyl glycol (dénomination INCI : Caprylyl glycol), l'adénosine (dénomination INCI : Adenosine), l'huile d'olive (dénomination INCI : Olea Europaea fruit oil) et le citrate de stéarate de glycéride (dénomination INCI : Glyceryl stéarate citrate).

La composition selon l'invention peut également comprendre des agents filmogènes comme le chitosan (dénomination INCI : Chitosan).

La composition selon l'invention peut également comprendre des agents nettoyants comme le décyl glucoside (dénomination INCI : Decyl glucoside) et le scétéaryl sulfate de sodium (dénomination INCI : Sodium cetearyl sulfate).

La composition selon l'invention peut également comprendre des agents antioxydants comme la vitamine E (dénomination INCI : Tocopherol) et l'ascorbyl glucoside (dénomination INCI : Ascorbyl glucoside).

La composition selon l'invention peut également comprendre des agents gélifiants comme le sodium dextran sulfate (dénomination INCI : Sodium dextran sulfate).

La composition selon l'invention peut également comprendre des agents parfumants comme le tetradecane (dénomination INCI : Tetradecane).

La description qui suit concerne des exemples de compositions selon l'invention.

### Exemple 1 : Crème de jour

**Tableau 1**

| Dénomination INCI | Pourcentage massique |
|---|---|
| Glycerin | 5,000 |
| Caprilic/Capric Tiglyceride | 5,000 |
| Polyglyceryl-4 Diisostearate / Polyhydroxystearate / Sebecate | 3,000 |
| Sucrose distearate | 2, 600 |
| Cetyl alcohol | 2, 600 |
| Tridecyl stéarate | 1,320 |
| Sucroce stéarate | 1,500 |
| Xanthane gum | 1,000 |
| Butylen glycol | 3,000 |
| Phytospingosine | 0,010 |
| Chitosan | 0,005 |
| Caulerpa lentillifera extract | 3, 100 |
| Hydrolysed Rice Bran protein | 0,400 |
| Hibiscus Esculentus Fruit Extract | 3,000 |
| Luffa Cylindrica Fruit/Leaf/Stem Extract | 3,000 |
| Sodium benzoate | 0,300 |
| Citric acid | 0,100 |
| Potassium sorbate | 0,200 |
| Sodium hydroxide | 0,001 |
| Fragrance | 0,001 |
| Aqua | 64,863 |

### Exemple 2 : Tonic

**Tableau 2**

| Dénomination INCI | Pourcentage massique |
|---|---|
| Cetearyl alcohol | 5 |
| Caprilic/Capric Tiglyceride | 5 |
| Dicaprylyl carbonate | 1,5 |
| Glyceryl stéarate | 2 |
| Decyl glucoside | 3 |
| Butylène glycol | 1 |
| Glycerin | 3 |
| Propanediol | 1 |
| Sodium cetearyl sulfate | 0,5 |
| Sodium benzoate | 0,3 |
| Citric acid | 0,1 |
| Caulerpa lentillifera extract | 0,1 |
| Hydrolysed Rice Bran protein | 0, 01 |
| Hibiscus Esculentus Fruit Extract | 0,1 |
| Luffa Cylindrica Fruit/Leaf/Stem Extract | 0,1 |
| Potassium sorbate | 0,2 |
| Tocopherol | 0, 01 |
| 1,2-Hexanediol | 0,3 |
| Caprylyl glycol | 0, 01 |
| Camelia sinensis leaf extract | 1 |
| Aqua | 75, 77 |

### Exemple 3 : Sérum

**Tableau 3**

| Dénomination INCI | Pourcentage massique |
|---|---|
| Xanthan gum | 0, 6 |
| Glycerin | 3 |
| 1,2-Hexandiol | 1 |
| Butylène glycol | 3 |
| Adenosine | 0, 05 |
| Cetyl alcohol | 2 |
| Olea Europaea fruit oil | 1 |
| Sodium dextran sulfate | 0,4 |
| Sodium hyaluronate | 0, 05 |
| Sodium benzoate | 0,3 |
| Ascorbyl glucoside | 0,5 |
| Caulerpa lentillifera extract | 4 |
| Hydrolysed Rice Bran protein | 0,5 |
| Hibiscus Esculentus Fruit Extract | 4 |
| Luffa Cylindrica Fruit/Leaf/Stem Extract | 4 |
| Potassium sorbate | 0,2 |
| Heliantus Annuus seed oil | 1 |
| Tocopherol | 0,1 |
| Sodium hydroxide | 0, 01 |
| Camelia sinensis leaf extract | 2 |
| Caprylyl glycol | 1 |
| Aqua | 71,29 |

### Exemple 4 : Baume

**Tableau 4**

| Dénomination INCI | Pourcentage massique |
|---|---|
| Candelilla / Jojoba / Rice bran polyglyceryl-3 esters, Glyceryl stéarate, Cetearyl alcohol, Sodium stearoyl lactylate | 4 |
| Isoamyl cocoate | 2 |
| Tetradecane | 3 |
| Coco-caprylate/caprate | 2 |
| Cetyl ricinoleate | 2 |
| Cetyl alcohol | 1 |
| Propandiol | 3 |
| Butylène glycol | 3 |
| Lysolecithin / Sclerotium gum / Pullulan | 2 |
| Sodium benzoate | 0,3 |
| Citric acid | 0,1 |
| Caulerpa lentillifera extract | 6 |
| Hydrolysed Rice Bran protein | 0,23 |
| Hibiscus Esculentus Fruit Extract | 2 |
| Luffa Cylindrica Fruit/Leaf/Stem Extract | 5 |
| Potassium sorbate | 0,2 |
| Fragrance | 0, 05 |
| Aqua | 64, 12 |

### Exemple 5 : Crème de nuit

**Tableau 5**

| Dénomination INCI | Pourcentage massique |
|---|---|
| Glycerin | 4 |
| Sclerotium / Xanthan gum | 0,3 |
| Polyslycertyl-6 distearate / Jojoba esters / Polyglyceryl-3 beeswax / Cetyl alcohol | 4 |
| Glyceryl stéarate citrate | 0,3 |
| Olus oil | 3 |
| Cetyl alcohol | 2 |
| Caprylyl/capric tryglyceride | 5 |
| Butylène glycol | 3 |
| Octyldodecanol | 5 |
| Sodium benzoate | 0,3 |
| Citric acid | 0,1 |
| Caulerpa lentillifera extract | 1 |
| Hydrolysed Rice Bran protein | 0,11 |
| Hibiscus Esculentus Fruit Extract | 6 |
| Luffa Cylindrica Fruit/Leaf/Stem Extract | 0,5 |
| Potassium sorbate | 0,2 |
| Aqua | 65,19 |

La composition selon l'invention peut être une composition cosmétique ou dermatologique. Elle peut se présenter sous la forme d'un gel, d'une lotion, d'une mousse, d'un baume ou d'un sérum.

## Revendications

1. Composition cosmétique comprenant à titre de principe actif de l'extrait de Caulerpa lentillifera, des protéines de riz hydrolysées, de l'extrait d'Hibiscus esculentus Moench et de l'extrait de Luffa cylindrica, le reste de la composition comprenant des ingrédients généralement admissibles dans le domaine cosmétique.

2. Composition selon la revendication 1, **caractérisée en ce qu'**elle comprend entre 0,05% et 8% en masse d'extrait de Caulerpa lentillifera, entre 0,001% et 1% en masse de protéines de riz hydrolysées, entre 0,05% et 8% en masse d'extrait d'Hibiscus esculentus et entre 0,001% et 5% d'extrait de Luffa cylindrica.

3. Composition selon la revendication 1 ou 2, **caractérisée en ce qu'**elle comprend également un ou plusieurs agents antioxydants.

4. Composition selon l'une quelconque des revendications précédentes, **caractérisée en ce qu'**elle comprend également de l'extrait de feuille de thé.

5. Composition selon la revendication 4, **caractérisée en ce qu'**elle comprend entre 1% et 3% d'extrait de feuille de thé.

6. Composition selon l'une quelconque des revendications précédentes, **caractérisée en ce qu'**elle se présente sous la forme d'une crème, d'un sérum, d'un gel, d'un baume ou d'une lotion.

## Patentansprüche

1. Kosmetische Zusammensetzung, welche als Wirkstoff einen Extrakt aus Caulerpa lentillifera, hydrolisierte Reisproteine, einen Extrakt aus Hibiscus esculentus Moench und einen Extrakt aus Luffa cylindrica umfasst, wobei der Rest der Zusammensetzung im Bereich der Kosmetik im Allgemeinen zulässige Inhaltsstoffe umfasst.

2. Zusammensetzung nach Anspruch 1, **dadurch gekennzeichnet, dass** sie zwischen 0,05 Ma.-% und 8 Ma.-% eines Extrakts aus Caulerpa lentillifera, zwischen 0,001 Ma.-% und 1 Ma.-% an hydrolisierten Reisproteinen, zwischen 0,05 Ma.-% und 8 Ma.-% eines Extrakts aus Hibiscus esculentus und zwischen 0,001 Ma.-% und 5 Ma.-% eines Extrakts aus Luffa cylindrica umfasst.

3. Zusammensetzung nach Anspruch 1 oder 2, **dadurch gekennzeichnet, dass** sie auch ein oder mehrere Antioxidationsmittel umfasst.

4. Zusammensetzung nach einem der vorstehenden Ansprüche, **dadurch gekennzeichnet, dass** sie auch einen Teeblattextrakt umfasst.

5. Zusammensetzung nach Anspruch 4, **dadurch gekennzeichnet, dass** sie zwischen 1% und 3% eines Teeblattextrakts umfasst.

6. Zusammensetzung nach einem der vorstehenden Ansprüche, **dadurch gekennzeichnet, dass** sie sich in Form einer Creme, eines Serums, eines Gels, eines Balsams oder einer Lotion darstellt.

## Claims

1. Cosmetic composition comprising as an active ingredient, Caulerpa lentillifera extract, hydrolysed rice proteins, Hibiscus esculentus Moench extract and Luffa cylindrica extract, the remainder of the composition comprising ingredients which are generally allowable in the cosmetic field.

2. Composition according to claim 1, **characterised in that** it comprises between 0.05% and 8% by weight Caulerpa lentillifera extract, between 0.001% and 1% by weight hydrolysed rice proteins, between 0.05% and 8% by weight Hibiscus esculentus extract and between 0.001% and 5% Luffa cylindrica extract.

3. Composition according to claim 1 or 2, **characterised in that** it also comprises one or more antioxidant agents.

4. Composition according to any one of the preceding claims, **characterised in that** it also comprises tea leaf extract.

5. Composition according to claim 4, **characterised in that** it comprises between 1% and 3% tea leaf extract.

6. Composition according to any one of the preceding claims, **characterised in that** it is in the form of a cream, a serum, a gel, a balm or a lotion.
